# EUROPEAN PATENT APPLICATION

(11) **EP 3 318 875 A1**
(43) Date of publication of application: **09.05.2018**
(21) Application number: 16817990.1
(22) Date of filing: 29.06.2016
(51) Int. Cl.: G01N 33/543, G01N 33/558

(54) **CHROMATOGRAPHIC ANALYSIS DEVICE AND CHROMATOGRAPHIC ANALYSIS METHOD**

(30) Priority: 30.06.2015 JP 2015131804
(71) Applicant: Tanaka Kikinzoku Kogyo K.K., Chiyoda-ku Tokyo 100-6422 (JP)
(72) Inventor: SUZUKI, Keita, Hiratsuka-shi Kanagawa 254-0076 (JP); IWAMOTO, Hisahiko, Hiratsuka-shi Kanagawa 254-0076 (JP)
(74) Representative: Peguet, Wilfried
(86) International application number: PCT/JP2016/069343
(87) International publication number: WO 2017/002882

(57) **Abstract**

The present invention provides a chromatographic analysis device capable of increasing the detection sensitivity to a detection target while suppressing a nonspecific reaction. The present invention relates to a chromatographic analysis device which includes at least a chromatographic medium part (3) on which a detection part (4) for detecting a detection target contained in an analyte is supported, wherein the chromatographic medium part (3) has a structure in which a membrane is provided on a support, the average film thickness of the membrane is from 110 µm to 130 µm, and the developing flow rate of the membrane is from 30 to 45 sec/40 mm.

## Description

### TECHNICAL FIELD

The present invention relates to a chromatographic analysis device and a chromatographic analysis method.

### BACKGROUND ART

Recently, the importance of an immunoassay by immunochromatography which does not require the pretreatment of an analyte as a simple in vitro diagnostic kit or a portable diagnostic device for detecting an antigen in a sample solution utilizing a specific reactivity of an antibody has been increasing. In particular, a test kit for a pathogen such as a virus or a bacterium is an immunochromatographic analysis device which is familiar and widely used also in an ordinary hospital and clinic.

The simplest structure of the conventional immunochromatographic analysis device is a structure in which a sample addition part, a labeling substance retaining part, a chromatographic medium part on which a detection part is supported, and an absorption part for absorbing a liquid having passed through the detection part are mutually connected to each other.

With respect to such an immunochromatographic analysis device, an increase in sensitivity is required at present for detecting a small amount of an antigen such as an influenza virus.

Therefore, conventionally, a method in which the amount of an antibody to be bound to a labeling substance is increased, etc. has been performed. However, such a method has a problem that a nonspecific reaction is likely to occur. Meanwhile, when the amount of the antibody is decreased, sufficient sensitivity cannot be obtained.

In view of this, for example, Patent Document 1 proposes a technique in which a masking agent for metal ions is allowed to coexist in a reaction solution.

Patent Document 2 discloses a technique in which a control line for preventing diffusion by a capillary phenomenon of a biological material-containing solution is formed at a predetermined position, and the biological material-containing solution is dropped in the vicinity of the control line and localized.

However, in the conventional immuno chromatographic analysis device, the sensitivity could not be increased to a required level while suppressing a nonspecific reaction.

### RELATED ART DOCUMENTS

### PATENT DOCUMENTS

Patent Document 1: JP-A-H9-203735
Patent Document 2: JP-A-2009-264879

### DISCLOSURE OF THE INVENTION

### PROBLEMS TO BE SOLVED BY THE INVENTION

In view of this, an object of the present invention is to provide a chromatographic analysis device and a chromatographic analysis method capable of increasing the detection sensitivity to a detection target while suppressing a nonspecific reaction.

### MEANS FOR SOLVING THE PROBLEMS

The present invention is as follows.
1. A chromatographic analysis device, comprising: at least a chromatographic medium part on which a detection part for detecting a detection target contained in an analyte is supported, wherein the chromatographic medium part has a structure in which a membrane is provided on a support, the average film thickness of the membrane is from 110 µm to 130 µm, and the developing flow rate of the membrane is from 30 to 45 sec/40 mm.
2. The chromatographic analysis device according to above 1, wherein a sample addition part for adding the analyte, a labeling substance retaining part for retaining a labeling substance which recognizes the detection target contained in the analyte, and the chromatographic medium part are sequentially included.
3. The chromatographic analysis device according to above 1 or 2, wherein the membrane is a nitrocellulose membrane.
4. The chromatographic analysis device according to any one of above 1 to 3, wherein the analyte is at least one type selected from nasal discharge, sputum, saliva, a nasal swab, a pharyngeal swab, and feces.
5. The chromatographic analysis device according to any one of above 1 to 4, which is an immunochromatographic analysis device.
6. A chromatographic analysis method which uses the chromatographic analysis device according to any one of above 1 to 5, and comprises at least a step of detecting a detection target contained in the analyte by the detection part supported on the chromatographic medium part.
7. A chromatographic analysis method wherein the following steps (1) to (4) are sequentially performed using the chromatographic analysis device according to above 2:
   (1) a step of adding the analyte to the sample addition part,
   (2) a step of allowing a labeling substance retained in the labeling substance retaining part to recognize a detection target contained in the analyte,
   (3) a step of developing the analyte and the labeling substance in the chromatographic medium part as a mobile phase; and
   (4) a step of detecting the detection target in the developed mobile phase in the detection part.
8. The chromatographic analysis method according to above 7, wherein the membrane is a nitrocellulose membrane.
9. The chromatographic analysis method according to any one of above 6 to 8, wherein the analyte is at least one type selected from nasal discharge, sputum, saliva, a nasal swab, a pharyngeal swab, and feces.
10. The chromatographic analysis method according to any one of above 6 to 9, wherein the chromatographic analysis device is an immunochromatographic analysis device.

### EFFECTS OF THE INVENTION

According to the present invention, the average film thickness and the developing flow rate of the membrane in the chromatographic medium part are set within specific ranges, and therefore, the adverse effect of a substance causing a nonspecific reaction in the detection part can be maximally prevented, and also the detection sensitivity can be increased simultaneously.

### BRIEF DESCRIPTION OF DRAWINGS

[FIG. 1] FIG. 1 is a cross-sectional view for illustrating one example of the structure of a chromatographic analysis device.
[FIG. 2] FIG. 2 is a cross-sectional view for illustrating one example of the structure of a support and a membrane of a chromatographic medium part.
[FIG. 3] FIG. 3 is a scanning electron micrograph (SEM) of a cross section of a chromatographic medium part of Example 6.

### EMBODIMENTS FOR CARRYING OUT THE INVENTION

Hereinafter, embodiments for carrying out the present invention will be described in more detail.

The chromatographic analysis device and the chromatographic analysis method of the present invention are not particularly limited as long as they utilize specific binding based on biomolecular affinity. For example, immunochromatographic analysis device and method utilizing binding between an antigen and an antibody, nucleic acid chromatographic analysis device and method utilizing hybridization of a nucleic acid, etc. can be exemplified, and the chromatographic analysis device and the chromatographic analysis method of the present invention can be, other than these, chromatographic analysis device and method utilizing binding between a sugar and lectin, binding between a hormone and a receptor, binding between an enzyme and an inhibitor, or the like.

Hereinafter, the present invention will be further described by showing an immunochromatographic analysis device and an immunochromatographic analysis method preferred for the present invention, however, the present invention is not limited to the following embodiments.

The immunochromatographic analysis device of the present invention is not particularly limited as long as it includes at least a chromatographic medium part on which a detection part for detecting a detection target contained in an analyte is supported, and a membrane in the chromatographic medium part satisfies the below-mentioned average film thickness and developing flow rate, however, hereinafter, one embodiment of a preferred immunochromatographic analysis device of the present invention will be described with reference to the drawings.

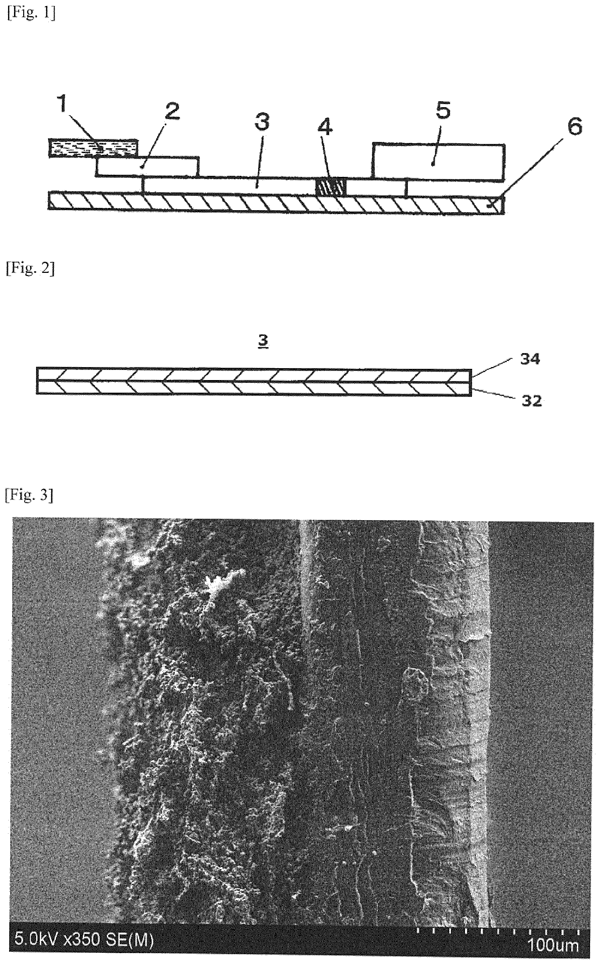

As shown in FIG. 1, the immunochromatographic analysis device of the present invention is configured to sequentially include a sample addition part (also referred to as "sample pad") (1), a labeling substance retaining part (also referred to as "conjugate pad") (2), a chromatographic medium part (3), a detection part (4), an absorption part (5), and a backing sheet (6).

The sample addition part (1) is a part where a sample which is an analyte is added in the immunochromatographic analysis device. The sample addition part (1) may be any as long as it is a material used for a common immunochromatographic analysis device. That is, the sample addition part (1) can be constituted by a porous sheet having a property such that a sample is promptly absorbed, but the sample promptly migrates therein. Examples of the porous sheet include a cellulose filter paper, glass fiber, polyurethane, polyacetate, cellulose acetate, nylon, and a cotton cloth.

The labeling substance retaining part (2) contains a labeling substance (marker substance), which will be described later, bound to an antibody which binds to a detection target in advance. The detection target is bound to the antibody and labeled with the antibody when the detection target migrates in the labeling substance retaining part. The labeling substance retaining part (2) is composed of, for example, a glass fiber non-woven fabric, a cellulose film, or the like.

The chromatographic medium part (3) is a developing part in a chromatograph. The chromatographic medium part (3) is an inactive film composed of a microporous material exhibiting a capillary phenomenon.

As shown in FIG. 2, the chromatographic medium part (3) has a structure in which a membrane (34) is provided on a support (32), and in the present invention, the average film thickness of the membrane (34) is from 110 µm to 130 µm, and the developing flow rate of the membrane (34) is from 30 to 45 sec/40 mm.

When the average film thickness of the membrane (34) is less than 110 µm, the membrane (34) is easily detached from the support (32) or easily scratched, and therefore, it is not practical. On the other hand, when the average film thickness of the membrane (34) exceeds 130 µm, a nonspecific reaction is likely to occur for an analyte having a high viscosity.

As for the thickness of the membrane (34), for example, a boundary line between the support (32) and the membrane (34) is specified by a scanning electron micrograph of the cross section of the chromatographic medium part (3) composed of the support (32) and the membrane (34) (for example, FIG. 3), and the thickness of the membrane (34) can be measured.

Further, the film thickness of the membrane (34) can also be calculated by measuring the film thickness of each of the chromatographic medium part (3) and the support (32) remaining after soaking the chromatographic medium part (3) in an organic solvent such as methanol to dissolve the membrane (34) using a commercially available film thickness meter such as a micrometer or a dial gauge, and obtaining a difference in film thickness before and after dissolving the membrane (34). By using these measurement methods, the measurement is performed at a plurality of different positions (preferably 9 to 10 or more positions) in the chromatographic medium part (3), and the average value thereof is calculated as the average film thickness of the membrane (34).

In order to suppress a nonspecific reaction with higher sensitivity, the membrane (34) having an average film thickness of 110 µm to 127 µm is preferably used, the membrane (34) having an average film thickness of 110 µm to 120 µm is more preferably used, and the membrane (34) having an average film thickness of 114 µm to 117 µm is most suitable. Further, when the thickness of the membrane (34) is within a range of 110 µm to 132 µm, the effect of the present invention can be sufficiently exhibited, and therefore is preferred, and the membrane (34) having an average film thickness of 110 µm to 122 µm is more preferably used.

When the developing flow rate of the membrane (34) is less than 30 sec/40 mm, it is difficult to obtain sufficient sensitivity, and on the other hand, when the developing flow rate exceeds 45 sec/40 mm, a nonspecific reaction is likely to occur.

The developing flow rate of the membrane (34) is more preferably from 35 to 45 sec/40 mm.

Incidentally, the developing flow rate in the present invention means a value obtained by measuring a time for which water is developed at a distance of 40 mm in the vertical direction on the membrane.

In general, when the average film thickness of the membrane (34) becomes thinner, the average pore diameter of the membrane (34) becomes smaller, and the developing flow rate becomes slower. On the other hand, when the average film thickness of the membrane (34) is increased, the developing flow rate becomes faster. In the present invention, as compared with a chromatographic analysis device of the conventional art, the average film thickness of the membrane (34) is set thinner, and also the developing flow rate is set faster. By setting the average film thickness of the membrane (34) thinner, the probability of contact between a detection target contained in an analyte and an antibody in the detection part (4) is increased, and therefore, the sensitivity can be increased, and also by setting the developing flow rate faster, the adverse effect of a substance causing a nonspecific reaction in the detection part (4) can be maximally prevented.

Incidentally, in order to set the average film thickness of the membrane (34) thinner and the developing flow rate faster, a method for adjusting the average pore diameter of the membrane (34) can be exemplified, and this adjustment of the average pore diameter can be performed by, for example, appropriately adjusting the amount of water contained in an organic solvent when a polymer constituting the membrane (34) is dissolved in a solution containing the organic solvent, followed by casting to form a membrane.

In the present invention, from the viewpoint of having no reactivity with a detection reagent, an immobilization reagent, a detection target, and the like to be used in the chromatograph and also from the viewpoint of enhancing the effect of the present invention, for example, a membrane made of nitrocellulose (hereinafter sometimes referred to as "nitrocellulose membrane") or a membrane made of cellulose acetate (hereinafter sometimes referred to as "cellulose acetate membrane") is preferred, and a nitrocellulose membrane is more preferred. Incidentally, it is also possible to use a cellulose-based membrane, a nylon membrane, and a porous plastic cloth (polyethylene or polypropylene).

The nitrocellulose membrane may be any as long as it contains nitrocellulose as a main component, and a membrane which contains nitrocellulose as a main material such as a pure product or a nitrocellulose mixed product can be used.

The nitrocellulose membrane exhibits a capillary phenomenon, however, a substance which promotes the capillary phenomenon can also be incorporated therein. As the substance, a substance which lowers the surface tension of the film surface to impart hydrophilicity is preferred. For example, a substance which has an amphiphilic action, does not affect the migration of the detection target on the immunochromatograph, and does not affect the development of the color of the labeling substance (for example, a gold particle or the like) such as a saccharide, an amino acid derivative, a fatty acid ester, or any of a variety of synthetic surfactants or alcohols is preferred.

The membrane (34) in the present invention has a thin average film thickness as described above, and therefore, in order to maintain its structure, the chromatographic medium part (3) has a structure in which the membrane (34) is provided on the support (32).

As the support (32), a support composed of a water-impermeable plastic or the like can be exemplified, and examples thereof include film-like supports made of polyethylene terephthalate, polyethylene, or polyurethane. Incidentally, in the case where observation of measurement results is performed by visual determination, the support (32) preferably has a color which is not similar to the color brought about by the labeling substance, and is generally preferably colorless or white.

The thickness of the support (32) is, for example, from 50 µm to 130 µm, preferably from 80 µm to 120 µm.

The form and size of the chromatographic medium part (3) represented by a nitrocellulose membrane or a cellulose acetate membrane as described above are not particularly limited, and may be any as long as they are appropriate in terms of actual operation and observation of the results of the reaction.

The detection part (4) is formed on the chromatographic medium part (3), that is, an antibody which specifically binds to the detection target is immobilized at an arbitrary position.

Examples of the antibody include a polyclonal antibody and a monoclonal antibody. The monoclonal antibody and the polyclonal antibody or fragments thereof are known and available, and can be prepared by a known method. The antibody is immobilized at an arbitrary position as an immobilization reagent, whereby the detection part (4) as a reaction region can be formed.

As a method for immobilizing the immobilization reagent on the chromatographic medium part (3), there are a method in which the immobilization reagent is directly immobilized on the chromatographic medium part (3) through a physical or chemical means and an indirect immobilization method in which the immobilization reagent is physically or chemically bound to fine particles, and the fine particles are trapped in the chromatographic medium part (3).

In the direct immobilization method, physical adsorption may be utilized, or a covalent bond may be used. In the case of a nitrocellulose membrane, physical adsorption can be performed. In the case of using a covalent bond, in order to activate the chromatographic medium part (3), cyanogen bromide, glutaraldehyde, carbodiimide, or the like is generally used, however, any method can be used.

As the indirect immobilization method, there is a method in which the immobilization reagent is bound to insoluble fine particles, and thereafter immobilized on the chromatographic medium part (3). With respect to the particle diameter of the insoluble fine particles, fine particles having such a size that the fine particles are trapped in the chromatographic medium part (3) but cannot migrate therein can be selected, and are preferably fine particles having an average particle diameter of about 5 µm or less.

As the particles, various particles to be used for an antigen-antibody reaction are known, and also in the present invention, these known particles can be used. Examples thereof include fine particles of an organic polymeric substance such as latex particles of an organic polymer obtained by emulsion polymerization method such as polystyrene, a styrenebutadiene copolymer, a styrene-methacrylate copolymer, polyglycidyl methacrylate, or an acrolein-ethylene glycol dimethacrylate copolymer, fine particles of gelatin, bentonite, agarose, crosslinked dextran, or the like, and inorganic particles of an inorganic oxide such as silica, silica-alumina, or alumina or inorganic particles in which a functional group is introduced into an inorganic oxide by a silane-coupling treatment or the like.

In the present invention, from the viewpoint of ease of adjustment of sensitivity or the like, direct immobilization is preferred. Further, in the immobilization of the immobilization reagent on the chromatographic medium part (3), various methods can be used. For example, various techniques such as a microsyringe, a pen with an adjustment pump, and ink injection printing can be used. The form of the reaction region is not particularly limited, however, immobilization can also be performed in the form of a circular spot, a line extending in a direction perpendicular to the development direction in the chromatographic medium, a numeric character, a letter, a symbol such as + or -, or the like.

After the immobilization reagent is immobilized, in order to further prevent the decrease in the analysis accuracy due to a nonspecific reaction, according to need, a blocking treatment can be performed for the chromatographic medium part (3) by a known method. In general, in the blocking treatment, a protein such as bovine serum albumin, skim milk, casein, or gelatin is preferably used. After such a blocking treatment, according to need, for example, washing may be performed by using one surfactant or two or more surfactants in combination such as Tween 20, Triton X-100, or SDS.

The absorption part (5) is provided as needed for absorbing a liquid such as the analyte, the developing solution, etc. having passed through the detection part (4) at an end of the chromatographic medium part (3). In the immunochromatographic analysis device of the present invention, as the absorption part (5), for example, a material in which a polymer such as an acrylic acid polymer and a hydrophilic agent having an ethylene oxide group or the like are incorporated in glass fiber, pulp, cellulose fiber, or the like or a non-woven fabric thereof is used, and particularly preferably, glass fiber is used. When the absorption part (5) is composed of glass fiber, the backward migration of the sample solution can be greatly reduced.

The backing sheet (6) is a base material. By applying an adhesive to one surface or sticking an adhesive tape to one surface, the surface has adhesiveness, and on the adhesive surface, part or all of the sample addition part (1), the labeling substance retaining part (2), the chromatographic medium part (3), the detection part (4), and the absorption part (5) are provided in close contact with the surface. The backing sheet (6) is not particularly limited as the base material as long as it becomes impermeable to a sample solution and also is impermeable to moisture by the adhesive.

The immunochromatographic analysis method of the present invention is not particularly limited as long as it has at least a step of detecting a detection target contained in an analyte by the detection part (4) supported on the chromatographic medium part (3) using the above-mentioned immunochromatographic analysis device, however, it is preferred to sequentially perform the following steps (1) to (4).

(1) a step of adding the analyte to the sample addition part
(2) a step of allowing a labeling substance retained in the labeling substance retaining part to recognize the detection target contained in the analyte
(3) a step of developing the analyte and the labeling substance in the chromatographic medium part as a mobile phase
(4) a step of detecting the detection target in the developed mobile phase in the detection part

Hereinafter, the respective steps will be described.

### (1) Step of adding the analyte to the sample addition part

In the step (1), in the first place, an analyte-containing solution is preferably prepared by adjusting or diluting an analyte with an analyte dilution solution to such a concentration that the analyte migrates smoothly in the immunochromatographic medium part (3) without decreasing the measurement accuracy. In the second place, the analyte-containing solution is dropped on the sample addition part (1) in a predetermined amount (generally from 0.1 to 2 mL). When the analyte-containing solution is dropped, the analyte-containing solution starts to migrate in the sample addition part (1).

This analyte dilution solution can also be used as a developing solution, however, in general, water is used as a solvent, and thereto, a buffer solution, a salt, and a nonionic surfactant, and further, for example, one type or two or more types of proteins, polymeric compounds (PVP, etc.), ionic surfactants or polyanions, or antimicrobial agents, chelating agents, etc. for promoting an antigen-antibody reaction or suppressing a nonspecific reaction may be added. In the case where it is used as a developing solution, a mixture obtained by mixing an analyte and the developing solution in advance can be supplied and dropped on the sample addition part to effect development, or an analyte is supplied and dropped on the sample addition part first, and thereafter, the developing solution may be supplied and dropped on the sample addition part to effect development.

Examples of the analyte containing a detection target include biological samples, that is, nasal discharge, sputum, saliva, a nasal swab, a pharyngeal swab, feces, whole blood, serum, plasma, urine, spinal fluid, amniotic fluid, nipple discharge fluid, tear, sweat, exudates from the skin, and extracts from tissues, cells, and feces, and in addition thereto, milk, eggs, wheat, beans, beef, pork, chicken, and extracts from foods and the like containing the same.

Among these, in the present invention, the analyte is preferably at least one type selected from nasal discharge, sputum, saliva, a nasal swab, a pharyngeal swab, and feces. That is, the analyte is preferably a so-called highly viscous analyte. Such a highly viscous analyte contains a viscous substance such as mucin, and it is known that such a viscous substance is likely to cause a nonspecific reaction.

In the present invention, since the average film thickness of the membrane (34) is set within a range of 110 µm to 130 µm and the developing flow rate of the membrane (34) is set within a range of 30 to 45 sec/40 mm as described above, the probability of contact between a detection target contained in an analyte and an antibody in the detection part (4) is increased, and therefore, the sensitivity can be increased, and also by setting the developing flow rate faster, the arrival of the highly viscous substance in the detection part (4) can be delayed, and thus, a nonspecific reaction can be maximally prevented.

Specific examples of the detection target include carcinoembryonic antigen (CEA), HER2 protein, prostate specific antigen (PSA), CA19-9, α-fetoprotein (AFP), immunosuppressive acidic protein (IPA), CA15-3, CA125, estrogen receptor, progesterone receptor, fecal occult blood, troponin I, troponin T, CK-MB, CRP, human chorionic gonadotropin (hCG), luteinizing hormone (LH), follicle-stimulating hormone (FSH), syphilis antibody, influenza virus, RS virus, human hemoglobin, chlamydia antigen, group A β-hemolytic streptococcal antigen, HBs antibody, HBs antigen, rotavirus, adenovirus, albumin, and glycated albumin, however, the detection target is not limited thereto.

Preferably, fecal occult blood, troponin I, troponin T, CK-MB, CRP, influenza virus, RS virus, human hemoglobin, chlamydia antigen, group A β-hemolytic streptococcal antigen, HBs antibody, HBs antigen, rotavirus, adenovirus, albumin, or glycated albumin is used as the detection target.

### (2) Step of allowing a labeling substance retained in the labeling substance retaining part to recognize the detection target contained in the analyte

The step (2) is a step in which the analyte-containing solution added to the sample addition part in the step (1) is migrated to the labeling substance retaining part (2), and the labeling substance retained in the labeling substance retaining part is allowed to recognize the detection target in the analyte.

The labeling substance labels the antibody. In the labeling of a detection reagent in the immunochromatographic analysis method, in general, an enzyme or the like is also used, however, it is preferred to use an insoluble carrier as the labeling substance because it is suitable for visually determining the presence of the detection target. The labeled detection reagent can be prepared by sensitizing the antibody to the insoluble carrier. A method for sensitizing the antibody to the insoluble carrier may be performed in accordance with a known method.

As the insoluble carrier to serve as the labeling substance, metal particles such as gold, silver, or platinum, metal oxide particles such as iron oxide, non-metal particles such as sulfur, latex particles composed of a synthetic polymer, or other insoluble carrier can be used. The insoluble carrier is the labeling substance suitable for visually determining the presence of the detection target, and is preferably a colored substance in order to facilitate the visual determination. The metal particles and the metal oxide particles exhibit a specific natural color per se according to the particle diameter, and the color can be utilized as a label.

In particular, gold particles are preferred because detection is simple and easy, are hardly aggregated, and are less likely to cause nonspecific color development. The average particle diameter of the gold particles is, for example, from 10 nm to 250 nm, preferably from 35 nm to 120 nm. The average particle diameter can be obtained by randomly measuring the projected area equivalent circle diameters of 100 particles using a projected image taken by a transmission electron microscope (TEM, JEM-2010, manufactured by JEOL Ltd.), and calculating the average of the projected area equivalent circle diameters.

### (3) Step of developing the analyte and the labeling substance in the chromatographic medium part as a mobile phase

The step (3) is a step in which after the detection target is recognized by the labeling substance in the labeling substance retaining part in the step (2), the analyte and the labeling substance are allowed to pass on the chromatographic medium part as a mobile phase.

### (4) Step of detecting the detection target in the developed mobile phase in the detection part

The step (4) is a step in which the detection target in the analyte having passed on the chromatographic medium part as the mobile phase is specifically reacted and bound so as to be sandwiched like a sandwich by the labeling reagent and the antibody retained in the detection part, that is, supported and fixed thereto, by an antigen-antibody specific binding reaction, and the detection part is colored.

In the case where the detection target is not present, the labeling reagent dissolved in an aqueous component of the sample does not cause a specific binding reaction even if it passes through the detection part on the chromatographic medium part, and therefore, the detection part is not colored.

Finally, the aqueous component of the analyte-containing solution migrates to the absorption part (5).

Incidentally, in the above description, the immuno chromatographic analysis device and the immunochromatographic analysis method are described as an example, however, the present invention is not limited to the above-mentioned embodiments, and the effect can be obtained in any case as long as it utilizes specific binding based on biomolecular affinity.

### EXAMPLES

Hereinafter, the present invention will be further described by way of Examples, however, the present invention is not limited to the following examples.

### (1) Preparation of sample addition part

As a sample addition part, a non-woven fabric composed of glass fiber (manufactured by Millipore, Inc., 300 mm × 30 mm) was used.

### (2) Preparation of labeling substance retaining part

To 0.5 mL of a colloidal gold suspension (manufactured by Tanaka Kikinzoku Kogyo K.K., LC 40 nm), 0.1 mL of a solution of a mouse-derived anti-RS virus monoclonal antibody diluted with a phosphate buffer solution (pH 7.4) to a concentration of 0.05 mg/mL was added, and the resulting mixture was left to stand at room temperature for 10 minutes.

Subsequently, 0.1 mL of a phosphate buffer solution (pH 7.4) containing 1 mass% bovine serum albumin (BSA) was added thereto, and the resulting mixture was left to stand at room temperature for an additional 10 minutes. Thereafter, the mixture was thoroughly stirred, and then centrifuged at 8000 × g for 15 minutes. After removing the supernatant, 0.1 mL of a phosphate buffer solution (pH 7.4) containing 1 mass% BSA was added thereto. According to the above-mentioned procedure, a labeling substance solution was prepared.

A solution obtained by adding 300 µL of a 10 mass% aqueous solution of trehalose and 1.8 mL of distilled water to 300 µL of the above-prepared labeling substance solution was added uniformly to a 15 mm × 300 mm glass fiber pad (manufactured by Millipore, Inc.), followed by drying in a vacuum dryer, whereby a labeling substance retaining part was prepared.

### (3) Preparation of chromatographic medium part and detection part

On a support made of polyethylene terephthalate and having a thickness of 100 µm, a membrane (300 mm × 25 mm) made of nitrocellulose and having a thickness and a developing flow rate as shown in the following Table 1 and Table 2 was stacked, whereby a chromatographic medium part was formed. The thickness of the membrane shown in Table 1 and Table 2 was obtained as follows. After the below-mentioned immunochromatographic analysis device was prepared (cut), the film thickness was measured at arbitrary 3 positions in cross-sectional observation of each of arbitrary 3 regions in a scanning electron micrograph of the chromatographic medium part in the immunochromatographic analysis device, and the minimum value of the film thickness, the maximum value of the film thickness, and the average value of the film thickness (average film thickness) of the chromatographic medium were calculated from the measured film thicknesses at 9 positions in total.

Subsequently, 150 µL of a solution obtained by diluting a mouse-derived anti-RS virus monoclonal antibody with a phosphate buffer solution (pH 7.4) containing 5 mass% isopropyl alcohol to a concentration of 1.0 mg/mL was applied to a detection region (detection line) with a width of 1 mm on the dried membrane, and in order to confirm the presence or absence of development of a gold nanoparticle labeling reagent or the developing rate, on the downstream of the detection region, a solution obtained by diluting a goat-derived antiserum having affinity in a wide range for the gold nanoparticle labeling substance and the like with a phosphate buffer solution (pH 7.4) was applied to a control region (control line). Thereafter, the solution was dried at 50°C for 30 minutes, and then dried at room temperature overnight, whereby a detection part was prepared on a chromatographic medium part.

### (4) Preparation of immunochromatographic analysis device

Subsequently, to a base material composed of a backing sheet, the sample addition part, the labeling substance retaining part, the chromatographic medium part having the detection part supported thereon, and a non-woven cloth made of glass fiber as an absorption part for absorbing the developed sample and labeling substance were sequentially bonded. Then, the resulting material was cut to a width of 5 mm by a cutting machine, whereby an immunochromatographic analysis device was prepared. The length in the direction of developing the sample of the labeling substance retaining part was set to 8 mm.

### (5) Analyte dilution solution

A 50 mM HEPES buffer solution (pH 7.5) containing 1 mass% nonionic surfactant (a 1:1 mixture of MN-811 (trade name), manufactured by NOF Corporation and NP-40 (trade name), manufactured by Nacalai Tesque, Inc.), 80 mM potassium chloride, 20 mM guanidine hydrochloride, and 0.4 wt% polyvinylpyrrolidone (average molecular weight: 360,000) was prepared and used as a reagent for performing a dilution treatment of an analyte.

### (6) Measurement

A nasal discharge which is an RS virus-negative clinical analyte was diluted to a concentration of 10% with the above-mentioned analyte dilution solution, and the resulting material was used as an analyte. This analyte in an amount of 120 µL was placed on the sample addition part of the immunochromatographic analysis device and developed, and after 15 minutes, the degree of color development in the detection part was confirmed by visual observation. The presence or absence of a nonspecific reaction was evaluated according to the following evaluation criteria.
++: A clear line was confirmed.
+: A light line was confirmed.
-: No line was confirmed.

Incidentally, a material obtained by diluting a nasal discharge which is an RS virus-positive clinical analyte to a concentration of 10% with the above-mentioned analyte dilution solution was used as an analyte, and the sensitivity was evaluated by repeating the above-mentioned measurement. The measurement was performed 5 times for each embodiment, and the result of average sensitivity is shown in Table 1 and Table 2.

### [Table 1]

**Table 1**

| | Example 1 | Example 2 | Example 3 | Example 4 | Example 5 | Example 6 |
|---|---|---|---|---|---|---|
| Thickness of membrane Minimum value (µm) | 125 | 127 | 121 | 112 | 112 | 110 |
| Thickness of membrane Maximum value (µm) | 132 | 132 | 129 | 122 | 121 | 118 |
| Thickness of membrane Average film thickness (µm) | 127 | 127 | 126 | 116 | 117 | 114 |
| Sensitivity (positive) | ++ | ++ | ++ | ++ | ++ | ++ |
| Nonspecific reaction (negative) | - | - | - | - | - | - |
| Developing flow rate (sec/40 mm) | 37 | 38 | 33 | 40 | 41 | 39 |

### [Table 2]

**Table 2**

| | Comparative Example 1 | Comparative Example 2 | Comparative Example 3 |
|---|---|---|---|
| Thickness of membrane Minimum value (µm) | 145 | 133 | 125 |
| Thickness of membrane Maximum value (µm) | 138 | 146 | 131 |
| Thickness of membrane Average film thickness (µm) | 141 | 141 | 127 |
| Sensitivity (positive) | ++ | ++ | + |
| Nonspecific reaction (negative) | + | + | - |
| Developing flow rate (sec/40 mm) | 42 | 39 | 54 |

In Table 1, it was found that in Examples 1 to 6, the average film thickness of the membrane was within a range of 110 µm to 130 µm, and also the developing flow rate of the membrane is within a range of 30 to 45 sec/40 mm, and therefore, even in the analysis using a highly viscous analyte, a nonspecific reaction does not occur. Further, also the sensitivity is favorable.

On the other hand, in Comparative Examples 1 and 2 in Table 2, although the developing flow rate is within the range of the present invention, the average film thickness of the membrane exceeds the upper limit defined in the present invention in both cases, and therefore, a nonspecific reaction occurred. In Comparative Example 3, although the average film thickness of the membrane is within the range of the present invention, the developing flow rate exceeds the upper limit defined in the present invention, and therefore, the sensitivity was lower than that of Examples.

Incidentally, in the case where the above-mentioned test was repeated by using only the analyte dilution solution as the analyte, both sensitivity and nonspecific reaction were evaluated as "-".

Further, in the case where with respect to each of Examples 1 to 6, the measurement was repeated 50 times, in Examples 4 to 6, a nonspecific reaction was not detected, however, in Examples 1 to 3, a nonspecific reaction was detected once or twice.

While the present invention has been described in detail with reference to specific embodiments, it is apparent to those skilled in the art that various changes and modifications can be made without departing from the spirit and scope of the present invention. The present application is based on Japanese Patent Application (Japanese Patent Application No. 2015-131804) filed on June 30, 2015 and the entire contents of which are incorporated herein by reference.

### REFERENCE SIGNS LIST

1. sample addition part (sample pad)
2. labeling substance retaining part
3. chromatographic medium part
32: support
34: membrane
4. detection part
5. absorption part
6. backing sheet

## Claims

1. A chromatographic analysis device, comprising:
at least a chromatographic medium part on which a detection part for detecting a detection target contained in an analyte is supported, wherein
the chromatographic medium part has a structure in which a membrane is provided on a support,
the average film thickness of the membrane is from 110 µm to 130 µm, and
the developing flow rate of the membrane is from 30 to 45 sec/40 mm.

2. The chromatographic analysis device according to claim 1,
wherein a sample addition part for adding the analyte, a labeling substance retaining part for retaining a labeling substance which recognizes the detection target contained in the analyte, and the chromatographic medium part are sequentially included.

3. The chromatographic analysis device according to claim 1 or 2,
wherein the membrane is a nitrocellulose membrane.

4. The chromatographic analysis device according to any one of claims 1 to 3,
wherein the analyte is at least one type selected from nasal discharge, sputum, saliva, a nasal swab, a pharyngeal swab, and feces.

5. The chromatographic analysis device according to any one of claims 1 to 4,
which is an immunochromatographic analysis device.

6. A chromatographic analysis method
which uses the chromatographic analysis device according to any one of claims 1 to 5, and comprises at least a step of detecting a detection target contained in the analyte by the detection part supported on the chromatographic medium part.

7. A chromatographic analysis method
wherein the following steps (1) to (4) are sequentially performed using the chromatographic analysis device according to claim 2:
(1) a step of adding the analyte to the sample addition part,
(2) a step of allowing a labeling substance retained in the labeling substance retaining part to recognize a detection target contained in the analyte,
(3) a step of developing the analyte and the labeling substance in the chromatographic medium part as a mobile phase; and
(4) a step of detecting the detection target in the developed mobile phase in the detection part.

8. The chromatographic analysis method according to claim 7,
wherein the membrane is a nitrocellulose membrane.

9. The chromatographic analysis method according to any one of claims 6 to 8,
wherein the analyte is at least one type selected from nasal discharge, sputum, saliva, a nasal swab, a pharyngeal swab, and feces.

10. The chromatographic analysis method according to any one of claims 6 to 9,
wherein the chromatographic analysis device is an immunochromatographic analysis device.
